# EUROPEAN PATENT APPLICATION

(11) **EP 4 831 157 A1**
(43) Date of publication of application: **28.10.2026**
(21) Application number: 25750032.2
(22) Date of filing: 24.03.2025
(51) Int. Cl.: C07C 279/28, C07C 277/08

(54) **METHOD FOR PREPARING CYANAMIDE PRODUCT**

(30) Priority: 03.03.2025 CN 202510243009
(71) Applicant: Sichuan Golden-Elephant Sincerity Chemical Co., Ltd., Meishan, Sichuan 620031 (CN); Sichuan Olefin Catalytic Materials Co., Ltd., Meishan, Sichuan 620031 (CN)
(72) Inventor: YU, Xiangzhen, Meishan, Sichuan 620031 (CN); TANG, Yin, Meishan, Sichuan 620031 (CN); FENG, Rui, Meishan, Sichuan 620031 (CN); WANG, Wenhong, Meishan, Sichuan 620031 (CN); LI, Gang, Meishan, Sichuan 620031 (CN); SONG, Guotian, Meishan, Sichuan 620031 (CN); KONG, Deli, Meishan, Sichuan 620031 (CN)
(74) Representative: Lavoix
(86) International application number: PCT/CN2025/084466
(87) International publication number: WO 2026/183894

(57) **Abstract**

The present application provides a method for preparing cyanamide-based products, including the following steps: S1. mixing raw material with a preheated solid catalyst, wherein the time when the raw material starts to contact the preheated solid catalyst is denoted as T₀, the time when reaches a uniform temperature after mixing is denoted as T₁, and T₁-T₀ ≤ 3s, the raw material comprises urea or mixed gas of ammonia gas and carbon dioxide, where the temperature of urea is less than or equal to 150 °C, and the temperature of the mixed gas ranges from 150 °C to 450 °C; S2. performing gas-solid separation on the obtained reaction mixture, wherein the time when the gas-solid separation is completed is denoted as T₂, and T₂-T₁ ≤ 10s; S3. cooling the obtained gas-phase product to 110 °C or less within F seconds, where F<1, to obtain the cyanamide-based products. Using the technical solution of the present application can enable monocyanamide and/or dicyandiamide have a higher selectivity, thereby making the content of monocyanamide and/or dicyandiamide in the finally obtained cyanamide-based products greater than 96%.

## Description

### CROSS REFERENCE TO RELATED APPLICATIONS

The present application claims priority to Chinese Patent Application No. 202510243009.3, filed for the China National Intellectual Property Administration (CNIPA) on March 3, 2025, entitled "Method for preparing cyanamide-based products", the entire contents of which are hereby incorporated by reference.

### TECHNICAL FIELD

The present application relates to the field of chemical industry, specifically to a method for preparing cyanamide-based products.

### BACKGROUND

Monocyandiamide, also called as cyanamide, urea anhydride, or aminonitrile, has a molecular formula of NH₂CN. The crystalline substance of monocyandiamide is not very stable and is often stored in a form of 30%-50% aqueous solution; under certain conditions, monocyandiamide can undergo polymerization and be transformed into dicyandiamide. Monocyandiamide is an important chemical industry raw material and has a wide range of uses, and it can be used for the synthesis of low toxicity and broad-spectrum chemical pesticide intermediates, health care products, feed additives, flame retardants, etc.

Dicyandiamide, also called as cyanoguanidine, dicyandiamide, dimeric cyanamide, has a chemical formula of C₂H₄N₄, which is a dimer of monocyandiamide and a cyano derivative of guanidine. Dicyandiamide is a white crystalline powder that is soluble in water, alcohol, ethylene glycol and dimethylformamide, and almost insoluble in ether and benzene. It is stable when dried and can be converted to melamine at high temperatures. Dicyandiamide, as a fine chemical industry intermediate, is used in medicine to prepare guanidine nitrate and sulfonamide drugs, and it can also be used to prepare thiourea, nitrocellulose stabilizers, rubber vulcanization accelerators, steel surface hardening agents, artificial leather fillers and adhesives, etc.

Currently, the calcium cyanamide method is the primary method used in the industrial production of monocyandiamide. The specific production process of calcium cyanamide method comprises: hydrolyzing calcium cyanamide to obtain a suspension of calcium hydrogen dicyanamide, removing the calcium hydroxide filter residue through vacuum filtration, and then introducing carbon dioxide into the filtrate to precipitate calcium in the form of calcium carbonate to obtain a monocyandiamide solution. Monocyanamide is polymerized under alkaline conditions, and then undergoes filtration, cooling crystallization, separation, and drying to obtain dicyandiamide.

The reaction equations are as follows:

2CaCN₂ + 2H₂O = Ca(HCN₂)₂ + Ca(OH)₂

Ca(HCN₂)₂ + CO₂ +H₂O = 2NH₂CN + CaCO₃

2NH₂CN = (NH₂CN)₂

This method involves a complex process, high equipment investment, severe pollution, and high energy consumption, and the product contains more free state calcium ions. Moreover, the production of calcium cyanamide would also generate a large amount of flue dust and waste heat, which not only pollutes the environment but also wastes resources. With the advancement of new pattern industrialization, the improvement of national resource comprehensive utilization, and environmental protection policies and regulations, the crude and extensive approach to producing deep-processing products of calcium carbide will be unsustainable.

Another way for preparing cyanamide-based products is to use urea or mixed gas of ammonia gas and carbon dioxide. Under the action of a catalyst, dehydration of urea or the mixed gas of ammonia gas and carbon dioxide produces monocyanamide, which then undergoes polymerization under certain conditions and is transformed into dicyandiamide. The reaction equations are as follows:

CO(NH₂)₂ = H₂O + NH₂CN(monocyandiamide)

CO₂ + 2NH₃ = 2H₂O + NH₂CN(monocyandiamide)

2NH₂CN(monocyandiamide) = (NH₂CN)₂(dicyandiamide)

Chinese patent document CN105984888A discloses a method for preparing monocyandiamide using urea as raw material. In this method, manganese acetate modified HZSM-5 molecular sieve is used as a catalyst, and ammonia gas is used as fluidized gas, urea particles are heated to 500-550 °C, which then are mixed with the catalyst heated to 500-550 °C for reaction. After absorbing ammonia and carbon dioxide in the products utilizing dilute sulfuric acid solution and sodium hydroxide solution, a cyanamide solution is obtained. The by-product yield of this process is high, and the selectivity of cyanamide is poor.

Chinese patent document CN113651725A discloses a method for preparing dicyandiamide by reusing melamine tail gas. The main constituents of melamine tail gas are ammonia and carbon dioxide. The product obtained after the reaction between ammonia and carbon dioxide is directly polymerized in a polymerizer, and then the product was cooled and crystallized in a crystallizer by heat exchange with water through a heat exchanger tube. However, this technology does not specify the product constituent, and dicyandiamide and melamine are not easy to separate.

In "Study of the new process for preparing cyanamide" (Wang Qian, Master's thesis, Tianjin University, 2006), urea or mixed gas of ammonia gas and carbon dioxide is heated and fed, ZSM-5 molecular sieve, Beta molecular sieve and aluminosilicate OX-50 zeolite are used as catalysts, the conversion rate could reach 88.5% or higher, but the product is a mixture of monocyanamide, dicyandiamide and melamine. Moreover, the melamine content is between 9.8% and 18.5% and the product is not easy to separate (see Table 2-3 in Chapter 2).

In conclusion, it can be seen that the existing technology uses the process for preparing cyanamide-based products by using urea or mixed gas of ammonia gas and carbon dioxide as raw materials, which commonly has the problems of excessive side reactions and poor product selectivity. Therefore, it is urgently needed to develop a method for preparing cyanamide-based products with few side reactions and high selectivity.

### SUMMARY OF THE INVENTION

In view of this, the present application intends to provide a method for preparing cyanamide-based products with few side reactions and high selectivity to solve the problems of excessive side reactions and poor product selectivity in existing processes.

According to the embodiments of the present application, the first aspect provides a method for preparing cyanamide-based products, comprising the following steps:
S1. mixing raw material with a preheated solid catalyst to initiate a reaction, wherein the time when the raw material starts to contact the preheated solid catalyst is denoted as T₀, the time when the raw material and the preheated solid catalyst reach a uniform temperature after mixing is denoted as T₁, and T₁-T₀ ≤ 3s;
   wherein the raw material comprises urea or mixed gas of ammonia gas and carbon dioxide, the temperature of urea is less than or equal to 150 °C, and the temperature of the mixed gas ranges from 150 °C to 450 °C;
S2. performing gas-solid separation on a reaction mixture obtained from S1, wherein the time when the gas-solid separation is completed is denoted as T₂, and T₂-T₁ ≤ 10s;
S3. cooling a gas-phase product obtained from S2 to 110 °C or less within F seconds, where F<1, to obtain the cyanamide-based products,
   wherein the cyanamide-based products comprise monocyandiamide (NH₂CN) and/or dicyandiamide (C₂H₄N₄), and
   the temperature of the preheated solid catalyst ranges from 500 °C to 750 °C.

It can be understood that the uniform temperature refers to the same temperature between the raw material and the preheated solid catalyst.

In the present application, the selectivity of monocyanamide or dicyandiamide reaches 96% or higher in the method for preparing cyanamide-based products. In the present application, the content of monocyandiamide or dicyandiamide in cyanamide-based products is 96% or higher.

In some alternative embodiments, T₁-T₀≤1s.

In some alternative embodiments, T₂-T₁≤6s.

In some alternative embodiments, F≤0.1.

In some alternative embodiments, 0.01s ≤ T₁-T₀ ≤ 0.5s.

In some alternative embodiments, 0.5s ≤ T₂-T₁ ≤ 3s.

In some alternative embodiments, 0.001 ≤ F ≤ 0.08.

In some alternative embodiments, the temperature of the mixed gas ranges from 150 °C to 350 °C.

In some alternative embodiments, the temperature of urea is less than or equal to 120 °C.

In some alternative embodiments, the particle diameter of the solid catalyst ranges from 30 µm to 200 µm. Solid catalysts with particle sizes ranging from 30 µm to 200 µm are used and can achieve cyclic flow under the action of a carrier gas.

In some alternative embodiments, a method for preparing the solid catalyst comprises: mixing an active component, a binder and deionized water, spray-drying and shaping at 250 °C to 450 °C, and then roasting at 400 °C to 650 °C for 30 minutes to 6 hours to obtain the solid catalyst.

A mass ratio of the active component to the binder is (5-70): (30-95).

In some alternative embodiments, the active component comprises at least one of active silica-alumina powder, ZSM-5 molecular sieve, beta molecular sieve, mordenite molecular sieve, Y molecular sieve and MCM-22 molecular sieve. By using the above active component, the conversion efficiency of the raw materials is higher, and the occurrence of side reactions is reduced, which is beneficial for the generation of monocyanamide or dicyandiamide.

In some alternative embodiments, the binder comprises at least one of silica sol, silica-alumina gel, aluminum phosphate gel, pseudoboehmite and kaolinite.

In some alternative embodiments, in S3, an absorbent is used to spray the gas-phase product, or the gas-phase product is brought into collision with the absorbent to achieve rapid cooling of the gas-phase product; the absorbent has a temperature ranging from 45 °C to 100 °C and a pH value ranging from 2.0 to 11.5. The adoption of methods that spraying the gas-phase product or colliding the gas-phase product with the absorbents can quickly cool the gas-phase product, reduce the generation of melamine, and thus increase the content of monocyanamide or dicyandiamide in the cyanamide-based products.

In some alternative embodiments, in S1, a riser process or a downer process is used to achieve rapid heating and reaction of the raw material;
wherein, in the riser process, the raw material and the catalyst enter from lower part of a reaction tube, while products and the catalyst exit from upper part of the reaction tube; in the downer process, the raw material and the catalyst enter from upper part of a reaction tube, while products and the catalyst exit from lower part of the reaction tube.

In some alternative embodiments, when the pH value of the absorbent is between 2.0 and 8.0, the content of monocyandiamide in the resulting cyanamide-based products is greater than 96%.

In some alternative embodiments, when the pH value of the absorbent is greater than 8.0, the content of dicyandiamide in the resulting cyanamide-based products is greater than 96%.

In some alternative embodiments, the absorbent comprises at least one of water, methanol, ethanol, acetone, n-butylamine, diethylamine, propylamine, phosphoric acid aqueous solution, tetraethylammonium hydroxide aqueous solution, tetrapropylammonium hydroxide aqueous solution, sodium hydroxide aqueous solution and potassium hydroxide aqueous solution.

Specific absorbents can lower the temperature of the gas-phase product faster, thereby avoiding the generation of melamine.

In some alternative embodiments, the mass content of monocyanamide or dicyandiamide in the cyanamide-based products is greater than 96%, and the mass content of melamine is less than 1%.

In some alternative embodiments, the preparation devices used to achieve the method for preparing cyanamide-based products comprises: a reaction tube, a catalyst storage tank, a heater, a cyclone separator, a rapid cooler and a feeding controller; wherein, the heater is used to preheat the solid catalyst from the catalyst storage tank; the reaction tube is used to mix and react the preheated solid catalyst with the raw material from the feeding controller; the cyclone separator is used for performing gas-solid separation on a reaction mixture to obtain the catalyst after the reaction and a gas-phase product; and the rapid cooler is used to cool down the gas-phase product.

In some alternative embodiments, the catalyst storage tank can also be used to receive the catalyst after the reaction.

In the preparation method, a heater is first utilized to preheat the solid catalyst in the catalyst storage tank, and then the preheated solid catalyst is directly contacted and mixed with urea raw material provided by the feeding controller in the reaction tube, so as to heat up urea rapidly (within 3 seconds) to a temperature of 400 °C to 620 °C, shorten the residence time of urea in the temperature range of 135 °C to 350 °C, reduce the decomposition-condensation of urea and reduce the occurrence of side reactions; subsequently, the heated urea undergoes dehydration reaction under the action of the catalyst, generating monocyanamide; under the action of the raw material carrier gas, the reaction mixture containing monocyanamide and catalyst enters the cyclone separator for rapid gas-solid separation to reduce the generation of side reactions; the separated solid catalyst is returned to the catalyst storage tank, and the reaction product, monocyanamide, enters the rapid cooler and is rapidly cooled after colliding directly with the absorbent or being sprayed by the absorbent to prevent the polymerization of monocyanamide into melamine, thus producing cyanamide-based products with high selectivity.

It can be understood that when using mixed gas of ammonia gas and carbon dioxide instead of urea as raw material, the mixed gas can be preheated utilizing a feeding controller, and then the preheated solid catalyst is contacted and mixed with the mixed gas provided by the feeding controller in a reaction tube to initiate a reaction and generate monocyanamide. The subsequent gas-solid separation and cooling processes are the same as above, so will not be repeated.

It should be stated that when the pH value of the absorbent is greater than 8.0, monocyanamide will undergoes dimerization to form dicyandiamide, and the final obtained product is primarily dicyandiamide. However, when the pH value of the absorbent is less than or equal to 8.0, monocyanamide will exist in a stable form, and the final obtained product is primarily monocyanamide.

The technical solution of the present application has the following advantages.

The method for preparing cyanamide-based products provided by the present application can effectively shorten the residence time of urea in the temperature range from 135 °C to 350 °C, reduce the decomposition-condensation of urea and reduce the occurrence of side reactions by controlling the temperature of the raw material and limiting the time for mixing the raw material and preheated solid catalyst to achieve uniform temperature to less than 3 seconds, that is, the time from uniform mixing to gas-solid separation is less than 10 seconds. Moreover, the rapid separation of the product after the reaction from the solid catalyst may further effectively reduce the generation of side reactions. Furthermore, the polymerization of monocyanamide to melamine may be suppressed by controlling the cooling rate of the reaction product within 1 second, thereby producing cyanamide-based products with high selectivity. In summary, using the technical solution of the present application may reduce the occurrence of side reactions, suppress the generation of melamine, and make monocyanamide and/or dicyandiamide have a higher selectivity, resulting in a monocyanamide and/or dicyandiamide content greater than 96% in the final obtained cyanamide-based products.

Further, the use of solid catalysts with particle sizes ranging from 30 µm to 200 µm allows for cyclic flow under the action of a carrier gas, which is beneficial for the progress of the reaction.

By using active silica-alumina powder, ZSM-5 molecular sieve, beta molecular sieve, mordenite molecular sieve, Y molecular sieve and MCM-22 molecular sieve as active components, the conversion efficiency of raw material is higher, and the occurrence of side reactions is reduced, which is beneficial for the generation of monocyanamide or dicyandiamide.

Further, the adoption of methods that spraying the gas-phase product or colliding the gas-phase product with the absorbents can quickly cool the gas-phase product, reduce the generation of melamine, and thus increase the content of monocyanamide or dicyandiamide in the cyanamide-based products.

Further, in the present application, monocyanamide has extremely high selectivity by controlling the pH of the absorbent to 2.0-8.0, resulting in a monocyanamide content of greater than 96% in the finally obtained cyanamide-based products.

Further, in the present application, dicyandiamide has extremely high selectivity by controlling the pH of the absorbent to be greater than 8.0, resulting in a dicyandiamide content of greater than 96% in the finally obtained cyanamide-based products.

### DESCRIPTION OF THE DRAWINGS

In order to explain the specific embodiments in the present application or technical solutions in existing technology more clearly, a brief introduction to the accompanying figures required for description of the specific embodiments or existing technology will be given below. It is obvious that the accompanying figures in the following description are some embodiments of the present application. For those skilled in the art, other accompanying figures can be obtained according to these accompanying figures without creative labor.

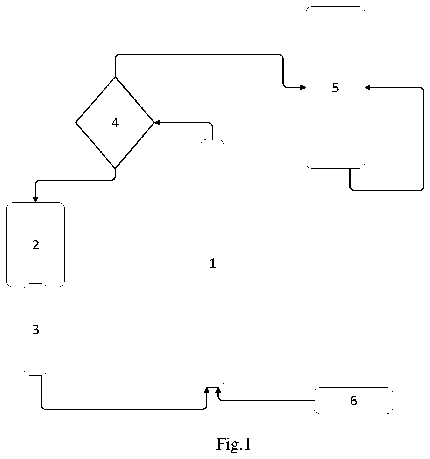

Fig. 1 is a system diagram of the method for preparing cyanamide-based products provided in Example 1 of the present application;
Accompanying figure illustration: 1-Reaction tube; 2-Catalyst storage tank; 3-Heater; 4-Cyclone separator; 5- Rapid cooler; 6-Feeding controller.

### DETAILED DESCRIPTION

The following examples are provided for a better understanding of the present application and are not limited to the best embodiment described. They do not limit the content and protection scope of the present application. Any product that is the same or similar to the present application which is derived from the inspiration of the present application or by combining the features of the present application with that of other existing technologies falls within the protection scope of the present application.

If specific experimental steps or conditions are not specified in the examples, the conventional experimental steps or conditions described in the literature in this field can be followed. If the manufacturer of the reagents or instruments used is not specified, they are all conventional reagents and products that can be purchased from the market.

### Example 1

The experimental device required for the preparation method of cyanamide-based products is shown as Figure 1, including: reaction tube 1, catalyst storage tank 2, heater 3, cyclone separator 4, rapid cooler 5 and feeding controller 6; wherein, heater 3 was used to preheat the solid catalyst in catalyst storage tank 2, and then the preheated solid catalyst was mixed and reacted with the raw material provided by the feeding controller 6 in reaction tube 1; the system after the reaction was sent to the cyclone separator 4 for separation of solid catalyst and reaction products, the separated solid catalyst was returned to the catalyst storage tank 2, and the reaction products were sent into the rapid cooler 5 for cooling to obtain the cyanamide-based products.

In a specific embodiment, reaction tube 1 was selected from a downer reactor, wherein the raw material enters from upper part of the downer reactor.

In a specific embodiment, reaction tube 1 was selected from a riser, where the raw material enters from lower part of the riser.

In a specific embodiment, the catalyst after the reaction was returned to the catalyst storage tank.

### Example 2

This example provides a method for preparing solid catalyst A, which is used in the preparation of cyanamide-based products, comprising the following steps.

500g of pseudoboehmite was taken and added into 1500g of deionized water, 350g of nitric acid with 65% concentration and 150g of phosphoric acid with 75% concentration were then added, under stirring, the slurry was heated to 80 °C and treated for 2 hours, 300g of kaolinite was then added and continued to be stirred for slurrying for 1h, and then 400g of H-type ZSM-5 molecular sieve was added. The prepared slurry was dried and molded by spray under 350 °C of hot air, and roasted at 500 °C for 2h to obtain catalyst A. A laser particle size analyzer (Zhuhai OMEC Instrument Co., Ltd., model LS-POP-9) was used to analyze, its particle size is in a range from 35 µm to 186 µm, and the average particle diameter (Dv50) is 83 µm.

### Example 3

This example provides a method for preparing cyanamide-based products, using the device provided in Example 1, comprising the following steps.

After all the air within the device was replaced with N₂, 6kg of catalyst A prepared according to the scheme in Example 2 was added into catalyst storage tank 2. With the assistance of N₂ gas, catalyst A was circulated in heater 3, reaction tube 1 (riser), cyclone separator 4 and catalyst storage tank 2, and heated by heater 3 to make catalyst A achieve a temperature of 550 °C. The circulation rate of catalyst A in reaction tube 1 was controlled at 3.5Kg/h.

In rapid cooler 5, the circulating absorbent was a phosphoric acid aqueous solution (i.e. deionized water was adjusted to pH 4.5 with phosphoric acid), the circulating flow rate is 6L/min, and the temperature was controlled at 50 °C.

After the preparation device was stabilized, urea at room temperature (25 °C) was added through the feeding controller 6 and sent into the reaction tube 1 utilizing ammonia gas at room temperature. The flow rate of urea was controlled at 2g/min, and the flow rate of ammonia gas was controlled at 50ml/min.

After the reaction was stable, the temperature of the reaction mixture at the outlet of reaction tube 1 was 450 °C. Then, the reaction mixture was sent into cyclone separator 4 for gas-solid separation, and the gas-phase product and catalyst B after the reaction was obtained. The gas-phase product entered rapid cooler 5 at a temperature of 400 °C, where the gas-phase product collided directly with the absorbent, thereby rapidly cooling the temperature of the gas-phase product to 50 °C; the catalyst B after the reaction was returned into the catalyst storage tank 2 for continued reuse.

After measurement and calculations, the time interval between T₁ and T₀ was 0.8s; the time interval between T₂ and T₁ was 4.5s; and the cooling time F for gas-phase product was 0.06s.

After 2 hours of stable operation, the liquid inside the rapid cooler was taken for analysis, and the analysis results are shown in Table 1.

After each test, all the liquid in rapid cooler 5 were replaced with new one.

### Example 4

This example provides a method for preparing cyanamide-based products, which is similar to Example 3 in steps, with the difference being the changes in the following parameters.

The circulation rate of catalyst A within reaction tube 1 was controlled at 3.8Kg/h.

The temperature was room temperature, the flow rate of urea was controlled at 2.5g/min, and the flow rate of ammonia gas was controlled at 60ml/min.

After the reaction was stable, the temperature of the reaction mixture at the outlet of reaction tube 1 (riser) was 480°C, and the temperature of the gas-phase product entered rapid cooler 5 was 420 °C.

After measurement and calculations, the time interval between T₁ and T₀ was 0.5s; the time interval between T₂ and T₁ was 3.0s; and the cooling time F for gas-phase product was 0.06s.

After 2 hours of stable operation, the liquid inside the rapid cooler was taken for analysis, and the analysis results are shown in Table 1.

### Example 5

This example provides a method for preparing cyanamide-based products, which is similar to Example 3 in steps, with the difference being the changes in the following parameters.

The catalyst A was heated up to a temperature of 600 °C by heater 3, and the catalyst circulation rate within reaction tube 1 was controlled at 3.8 Kg/h.

The flow rate of urea was controlled at 4.0g/min, and the flow rate of ammonia gas was controlled at 65ml/min.

After the reaction was stable, the temperature of the reaction mixture at the outlet of reaction tube 1 (riser) is 520 °C, and the temperature of the gas-phase product entered rapid cooler 5 was 450 °C.

After measurement and calculations, the time interval between T₁ and T₀ was 0.3s; the time interval between T₂ and T₁ was 2.2s; and the cooling time F for gas-phase product was 0.04s.

After 2 hours of stable operation, the liquid inside the rapid cooler was taken for analysis, and the analysis results are shown in Table 1.

### Example 6

This example provides a method for preparing cyanamide-based products, which is similar to Example 3 in steps, with the difference being the changes in the following parameters.

The catalyst A was heated up to a temperature of 580 °C by heater 3, and the circulation rate of catalyst A within reaction tube 1 was controlled at 3.0Kg/h. In rapid cooler 5, the circulating absorbent was tetraethylammonium hydroxide solution (i.e. deionized water was adjusted to pH 10.5 with tetraethylammonium hydroxide), the circulation flow rate was 8L/min, and the temperature was controlled at 95 °C.

After the device was stabilized, urea was added through the feeding controller 6 and sent into the reaction tube 1 using ammonia gas, the temperature of urea was room temperature, the flow rate of urea was controlled at 2.0g/min, and the flow rate of ammonia gas was controlled at 80ml/min.

After the reaction was stable, the temperature of the reaction mixture at the outlet of reaction tube 1 (riser) was 480 °C, and the temperature of the gas-phase product entered rapid cooler 5 was 410 °C.

After measurement and calculations, the time interval between T₁ and T₀ was 0.3s; the time interval between T₂ and T₁ was 3.6s; and the cooling time F for gas-phase product was 0.06s.

After 2 hours of stable operation, the circulating liquid was taken for analysis, and the analysis results are shown in Table 1.

### Example 7

This example provides a method for preparing cyanamide-based products, which is similar to Example 3 in steps, with the difference being the changes in the following parameters.

The catalyst A was heated up to 560 °C by heater 3, and the catalyst circulation rate within reaction tube 1 was controlled at 3.6 Kg/h. In rapid cooler 5, the absorbent was sodium hydroxide solution (i.e. deionized water was adjusted to pH 10.0 with sodium hydroxide), the circulation flow rate was 8 L/min, and the temperature was controlled at 100 °C.

After the device was stabilized, urea was added through the feeding controller 6 and sent into the reaction tube 1 using ammonia gas, the temperature was room temperature, the flow rate of urea was controlled at 3.0g/min, and the flow rate of ammonia gas was controlled at 85ml/min.

After the reaction was stable, the temperature of the reaction mixture at the outlet of reaction tube 1 (riser) was 450 °C, and the temperature of the gas-phase product entered rapid cooler 5 was 350 °C.

After measurement and calculations, the time interval between T₁ and T₀ was 0.3s; the time interval between T₂ and T₁ was 3.2s; and the cooling time F for gas-phase product was 0.08s.

After 2 hours of stable operation, the liquid inside the cooler was taken for analysis, and the analysis results are shown in Table 1.

### Example 8

This example provides a method for preparing cyanamide-based products, using the device provided in Example 1, comprising the following steps:
After replacing all the air within the device with N₂, 6.5Kg of catalyst A prepared according to the scheme in Example 1 was added into catalyst storage tank 2. With the assistance of N₂ gas, catalyst A was circulated in heater 3, reaction tube 1 (riser), cyclone separator 4 and catalyst storage tank 2, and heated by heater 3 to reach a catalyst temperature of 620 °C. The catalyst circulation rate in reaction tube 1 was controlled at 3.0Kg/h. In rapid cooler 5, the absorbent was phosphoric acid aqueous solution (i.e. deionized water was adjusted to pH 4.5 with phosphoric acid), the circulation flow rate was 8L/min, and the temperature was controlled at 50 °C.

After the catalyst A and the circulating absorbent achieves stabilized state in circulation, ammonia gas and carbon dioxide gas were introduced into the reaction tube 1 through the feeding controller 6, in which the gases were preheated to 350 °C, the flow rate of ammonia gas was controlled at 200 ml/min, and the flow rate of carbon dioxide gas was controlled at 3,000 ml/min.

After the reaction was stable, the temperature of the reaction mixture at the outlet of reaction tube 1 was 580 °C, the temperature of the gas-phase product entered rapid cooler 5 was 460 °C.

After measurement and calculations, the time interval between T₁ and T₀ was 0.1s; the time interval between T₂ and T₁ was 2.4s; and the cooling time F for gas-phase product was 0.06s.

After 2 hours of stable operation, the liquid inside the rapid cooler was taken for analysis, and the analysis results are shown in Table 1.

### Example 9

This example provides a method for preparing cyanamide-based products, using the device provided in Example 1, comprising the following steps.

After replacing all the air within the device with N₂, 6.5Kg of catalyst A prepared according to the scheme in Example 1 was added into catalyst storage tank 2. With the assistance of N₂ gas, catalyst A was circulated in heater 3, reaction tube 1 (riser), cyclone separator 4 and catalyst storage tank 2, and heated by heater 3 to reach a temperature of 600 °C. The catalyst circulation rate in reaction tube 1 was controlled at 3.6Kg/h. In rapid cooler 5, the absorbent was sodium hydroxide solution (i.e. deionized water was adjusted to pH 9.5 with sodium hydroxide), the circulation flow rate was 8L/min, and the temperature was controlled at 98 °C.

After the catalyst A and the circulating absorbent achieve stabilized state in circulation, ammonia gas and carbon dioxide gas were introduced into the reaction tube 1 through the feeding controller 6 and preheated to 350 °C, in which the flow rate of ammonia gas was controlled at 300ml/min and the flow rate of carbon dioxide gas was controlled at 6000ml/min.

After the reaction was stable, the temperature of the reaction mixture at the outlet of reaction tube 1 was 550 °C, and the temperature of the gas-phase product entered rapid cooler 5 was 430 °C.

After measurement and calculations, the time interval between T₁ and T₀ was 0.1s; the time interval between T₂ and T₁ was 3.0s; and the cooling time F for gas-phase product was 0.08s.

After 2 hours of stable operation, the circulating liquid was taken for analysis, and the analysis results are shown in Table 1.

### Comparative Example 1

This comparative example provides a method for preparing cyanamide-based products, which is similar to Example 3 in steps, with the difference being the changes in the following parameters.

Urea was added through the feeding controller 6 and preheated to 220 °C before entering the reaction tube.

After 2 hours of stable operation, the liquid inside the rapid cooler was taken for analysis, and the analysis results are shown in Table 1.

### Comparative Example 2

This comparative example provides a method for preparing cyanamide-based products, which is similar to Example 3 in steps, with the difference being the changes in the following parameters.

Catalyst A was heated with heater 3 to reach a temperature of 580 °C, and the circulation rate of catalyst A within reaction tube 1 (riser) was control at 3.0Kg/h. In rapid cooler 5, the circulating absorbent was tetraethylammonium hydroxide solution (i.e. deionized water was adjusted to pH 10.5 with tetraethylammonium hydroxide), the circulation flow rate was 8L/min, and the temperature was controlled at 95 °C.

Urea was added through the feeding controller 6 and preheated to 220 °C before entering the reaction tube.

After 2 hours of stable operation, the liquid inside the rapid cooler was taken for analysis, and the analysis results are shown in Table 1.

An Agilent high-performance liquid chromatography (1260 Infinity II) was used to analyze the constituents of the cyanamide-based products prepared in the examples and comparative examples of the present application. The chromatographic conditions were as follows: an ODS chromatographic column was used, column temperature was 50 °C, mobile phase was water and 0.25% ammonia water, pH value was controlled at 6.5 by HClO₄, and flow rate was 1.000mL/min; detection wavelength was 205nm; and the sample injection volume was 10 µL. The selectivity of different components was calculated based on the calibrated peak area. The results are shown in Table 1.

**Table 1. Product Analysis Data Sheet**

| Name | Raw material | Urea or ammonia conversion, % | Monocyanamide content, wt% | Dicyandiamide content, wt% | Melamine content, wt% |
|---|---|---|---|---|---|
| Example 3 | Urea | 98.30% | 97.80% | 1.43% | 0.45% |
| Example 4 | Urea | 99.10% | 96.50% | 2.77% | 0.30% |
| Example 5 | Urea | 99.80% | 98.20% | 1.05% | 0.40% |
| Example 6 | Urea | 100.00% | 1.20% | 97.62% | 0.80% |
| Example 7 | Urea | 100.00% | 1.10% | 97.84% | 0.65% |
| Example 8 | Ammonia gas + carbon dioxide | 95.50% | 97.60% | 1.78% | 0.34% |
| Example 9 | Ammonia gas + carbon dioxide | 92.50% | 1.20% | 97.90% | 0.60% |
| Comparativ e Example 1 | Urea | 97.60% | 68.20% | 3.50% | 1.60% |
| Comparativ e Example 2 | Urea | 99.50% | 1.30% | 67.3% | 1.20% |

From Table 1 it can be seen that, in the preparation method of the present application, the conversion of urea is 98.30% or higher, and when using ammonia gas and carbon dioxide as raw materials, the conversion of ammonia is 92.50% or higher; when preparing monocyanamide, the selectivity of monocyanamide is 96.50% or higher; when preparing dicyandiamide, the selectivity of dicyandiamide is 97.62% or higher; at the same time, the yield of melamine is well controlled (1.0% or less).

Obviously, the above examples are only examples provided for clear illustration, and not limitations on the embodiments. For those ordinary skilled in the relevant field, other forms of changes or modifications can be made based on the above description. It is not necessary and impossible to exhaustively list all embodiments here. The obvious changes or modifications arising from this are still within the protection scope of the present invention.

## Claims

1. A method for preparing cyanamide-based products, **characterized in that**, the method comprises the following steps:
S1. mixing raw material with a preheated solid catalyst to initiate a reaction, wherein the time when the raw material starts to contact the preheated solid catalyst is denoted as T₀, the time when the raw material and the preheated solid catalyst reach a uniform temperature after mixing is denoted as T₁, and T₁-T₀ ≤ 3s; and
wherein the raw material comprises urea or mixed gas of ammonia gas and carbon dioxide, the temperature of the urea is less than or equal to 150 °C, and the temperature of the mixed gas ranges from 150 °C to 450 °C;
S2. performing gas-solid separation on a reaction mixture obtained from S1, wherein the time when the gas-solid separation is completed is denoted as T₂, and T₂-T₁ ≤ 10s;
S3. cooling a gas-phase product obtained from S2 to 110 °C or less within F seconds, where F<1, to obtain the cyanamide-based products,
wherein the cyanamide-based products comprise monocyanamide (NH₂CN) and/or dicyandiamide (C₂H₄N₄), and
the temperature of the preheated solid catalyst ranges from 500 °C to 750 °C.

2. The method for preparing cyanamide-based products according to claim 1, **characterized in that**, T₁-T₀≤1s; and/or, T₂-T₁≤6s; and/or, F≤0.1_{∘}

3. The method for preparing cyanamide-based products according to claim 2, **characterized in that**, 0.01s ≤ T₁-T₀ ≤ 0.5s; and/or, 0.5s ≤ T₂-T₁ ≤ 3s; and/or, 0.001 ≤ F ≤ 0.08.

4. The method for preparing cyanamide-based products according to claim 1, **characterized in that**, the temperature of the mixed gas ranges from 150 °C to 350 °C;
and/or, the temperature of urea is less than or equal to 120 °C.

5. The method for preparing cyanamide-based products according to claim 1, **characterized in that**, the particle diameter of the solid catalyst ranges from 30 µm to 200 µm.

6. The method for preparing cyanamide-based products according to claim 5, **characterized in that**, a method for preparing the solid catalyst comprises: mixing an active component, a binder and deionized water, spray-drying and shaping at 250 °C to 450 °C, and then roasting at 400 °C to 650 °C for 30 minutes to 6 hours to obtain the solid catalyst;
a mass ratio of the active component to the binder is (5-70): (30-95);
and/or, the active component comprises at least one of active silica-alumina powder, ZSM-5 molecular sieve, beta molecular sieve, mordenite molecular sieve, Y molecular sieve and MCM-22 molecular sieve;
and/or, the binder comprises at least one of silica sol, silica-alumina gel, aluminum phosphate gel, pseudoboehmite and kaolinite.

7. The method for preparing cyanamide-based products according to claim 1, **characterized in that**, in S3, an absorbent is used to spray the gas-phase product, or the gas-phase product is brought into collision with the absorbent to achieve rapid cooling of the gas-phase product; and the absorbent has a temperature ranging from 45 °C to 100 °C and a pH value ranging from 2.0 to 11.5.

8. The method for preparing cyanamide-based products according to claim 1, **characterized in that**, in S1, a riser process or a downer process is used to achieve rapid heating and reaction of the raw material;
wherein, in the riser process, the raw material and the catalyst enter from lower part of a reaction tube, while products and the catalyst exit from upper part of the reaction tube; in the downer process, the raw material and the catalyst enter from upper part of a reaction tube, while products and the catalyst exit from lower part of the reaction tube.

9. The method for preparing cyanamide-based products according to claim 7, **characterized in that**, when the pH value of the absorbent is greater than 8.0, a content of dicyandiamide in the obtained cyanamide-based products is 96% or higher; and when the pH value of the absorbent is between 2.0 and 8.0, a content of monocyanamide in the obtained cyanamide-based products is 96% or higher;
and/or, the absorbent comprises at least one of water, methanol, ethanol, acetone, n-butylamine, diethylamine, propylamine, phosphoric acid aqueous solution, tetraethylammonium hydroxide aqueous solution, tetrapropylammonium hydroxide aqueous solution, sodium hydroxide aqueous solution and potassium hydroxide aqueous solution.

10. The method for preparing cyanamide-based products according to any one of claims 1 to 9, **characterized in that**, preparation devices used to achieve the method for preparing cyanamide-based products comprises: a reaction tube, a catalyst storage tank, a heater, a cyclone separator, a rapid cooler and a feeding controller; wherein, the heater is used to preheat the solid catalyst from the catalyst storage tank; the reaction tube is used to mix and react the preheated solid catalyst with the raw material from the feeding controller; the cyclone separator is used for performing gas-solid separation on a reaction mixture to obtain the catalyst after the reaction and a gas-phase product; and the rapid cooler is used to cool down the gas-phase product;
and/or, the catalyst storage tank is also used to receive the catalyst after the reaction.
